# EUROPEAN PATENT APPLICATION

(11) **EP 2 623 021 A1**
(43) Date of publication of application: **07.08.2013**
(21) Application number: 11828445.4
(22) Date of filing: 28.09.2011
(51) Int. Cl.: A61B 5/00

(54) **MOISTURE METER**

(30) Priority: 29.09.2010 JP 2010219965
(71) Applicant: Terumo Kabushiki Kaisha, Tokyo 151-0072 (JP)
(72) Inventor: KOYAMA, Miyuki, Ashigarakami-gun Kanagawa 259-0151 (JP); YOSHINO, Keisuke, Ashigarakami-gun Kanagawa 259-0151 (JP)
(74) Representative: Kremer, Simon Mark
(86) International application number: PCT/JP2011/005483
(87) International publication number: WO 2012/042879

(57) **Abstract**

Provided is a moisture meter that can easily measure a moisture content of a subject and can be effectively used as means for assisting the subject in appropriately regulating the moisture content. A moisture meter (1) for measuring a moisture content of a subject (M) includes: a probe-type moisture measuring unit (30) that is held in an armpit (R) of the subject (M) so as to measure a moisture content in the armpit (R) in order to measure the moisture content of the subject (M); and a temperature measuring unit (31) that is held in the armpit (R) of the subject (M) so as to measure the temperature of the subject (M). The moisture meter (1) further includes: a main body (10); a measuring unit holder (12) that is sandwiched in the armpit (R) while holding the probe-type moisture measuring unit (30) and the temperature measuring unit (31); and a display unit holder (12) that holds a display unit (20) that displays the measured moisture content of the subject (M) and the measured temperature of the subject (M).

## Description

### TECHNICAL FIELD

The present invention relates to a moisture meter that is held in the armpit of a subject to measure a moisture content in a living body.

### Background Art

It is important to measure a moisture content in the living body of a subject. Dehydration in the living body is a pathological condition in which the moisture content in the living body decreases, and is a symptom that develops frequently in daily life, and in particular, develops more often when a subject is doing an exercise or the atmospheric temperature is high because a large amount of water is excreted from the body as a result of perspiration or a temperature rise. In particular, since in many cases, older persons have a decreased ability to retain water in the living body, it is said that older persons are more likely to have dehydration than ordinary healthy persons.

In general, when a person gets older, the volume of muscles that store water decreases, the urinary volume increases due to a decrease in the kidney function, the ability to sense thirst in the mouth decreases due to dulled sensitivity, and the moisture content required for the cells decreases. When the dehydration is left without any treatment, the dehydration may cause and even develop into severe symptoms. Infants may also suffer from the same dehydration. Although the moisture content of infants generally large, the infants cannot appropriately appeal for the supply of water by themselves and may have dehydration since the persons who care the infants recognize it too late.

In general, it is said that a disorder in temperature regulation occurs when more than 2% of weight of moisture in the living body is lost. The disorder in temperature regulation causes such a vicious circle that it causes an increase in temperature, which in turn causes a reduction in the moisture content in the living body, and finally results in a pathological condition called a heat illness. Heat illnesses include heat cramps, heat exhaustion, heat strokes, which may sometimes cause organ disorder in the entire body. Thus, it is preferable to accurately detect dehydration to prevent such a danger that leads to heat illnesses.
As a device for detecting dehydration, an apparatus that measures a body impedance using such a device having handles to be held by both hands to calculate a moisture content from the measured impedance is known (see Patent Documents 1 to 3).
As another device for detecting dehydration, an oral moisture meter or the like that measures a moisture content in the mouth such as lingual mucosa, buccal mucosa or palate is known (see Patent Documents 4 to 6).
As a method for measuring moisture content in the skin, an in-vitro mass method, a Karl Fischer method, an in-vivo ATR spectroscopy, and a high-frequency impedance method and electrical conductivity method which are simpler in-vivo measurement methods are generally used.

### Citation List

### Patent Literature

Patent Literature 1: Japanese Patent Application Laid-open No. H11-318845
Patent Literature 2: Japanese Patent Publication No. 3977983
Patent Literature 3: Japanese Patent Publication No. 3699640
Patent Literature 4: W02004/028359
Patent Literature 5: Japanese Patent Application Laid-open No. 2001-170088
Patent Literature 6: Japanese Patent Application Laid-open No. 2005-287547

### Summary of Invention

### Technical Problem

However, the moisture meter that measures the body impedance using such a device having handles to be held by both hands to calculate the moisture content from the body impedance measures the impedance of the skin of the hand. Thus, the apparatus is likely to be influenced by the humidity of the skin, the volume of arm muscles, and the like and is not user-friendly because the apparatus is too tall for older persons or physically disabled persons so these persons have to stand up to get measurements.
It is generally known that the bioelectrical impedance value decreases as the temperature increases and the bioelectrical impedance value increases as the temperature decreases, that is, the bioelectrical impedance value (that is, the moisture content) changes as the temperature changes. However, since the conventional moisture meter calculates the body moisture content from the bioelectrical impedance value that is measured without taking the fact that the bioelectrical impedance value varies with the temperature into consideration, it is not possible to obtain an accurate body moisture content and to accurately detect dehydration. For example, when the body moisture content decreases and the temperature increases, the bioelectrical impedance value increases due to the decrease in the body moisture content whereas the bioelectrical impedance value decreases due to the increase in the temperature. Thus, the dehydration state may be not detected even if it is determined based on the body moisture content that is calculated from the bioelectrical impedance value. Thus, when measurement is performed according to the impedance method, although it is necessary to know the temperature of the subject, the impedance value is not corrected based on the measured temperature, or a warning that it is not possible to determine the accurate moisture content because the subject has a fever is not output.

In the oral moisture meter that measures a moisture content in the mouth such as lingual mucosa, buccal mucosa or palate, it is necessary to attach a replaceable cover for each subject to a portion that is directly inserted into the mouth in order to prevent contaminations between subjects. Thus, users may forget to replace and attach the cover, and the oral moisture meter is not user-friendly to older persons or physically disabled persons.

A dehydration state determining apparatus disclosed in Japanese Patent Publication No. 13977983 includes a temperature sensor that measure the temperature of the thumb, and the apparatus corrects a measured bioelectrical impedance based on the temperature and determines a dehydration state based on the corrected bioelectrical impedance value. Since the dehydration state is determined based on the bioelectrical impedance value with the temperature taken into consideration, the dehydration state can be determined more accurately, and the subject can accurately examine the dehydration state.
However, in this document, although the temperature is measured using the thumb, it is difficult to measure the temperature in the thumb, which is not a practical method.
Therefore, an object of the present invention is to provide a moisture meter that can easily measure a moisture content of a subject and can be effectively used as means for assisting the subject in appropriately regulating moisture content.

### Solution to Problem

A moisture meter according to the present invention is a moisture meter for measuring a moisture content of a subject, including: a probe-type moisture measuring unit that is held in an armpit of the subject so as to measure a moisture content in the armpit in order to measure the moisture content of the subject.
According to this configuration, the moisture meter can measure the moisture content of the subject easily and can be effectively used as means for assisting the subject in appropriately regulating the moisture content. The reason for selecting the armpit as the location of the living body where the moisture content of the subject can be appropriately measured using the moisture meter and measuring the moisture content in the living body of the subject is because the moisture content measured in the armpit R best reflects the moisture state of the entire living body of the subject.

Preferably, the moisture meter includes a temperature measuring unit that is held in the armpit of the subject so as to measure the temperature of the subject.
According to this configuration, by measuring the temperature of the subject simultaneously with measuring the moisture content of the subject in the armpit of the subject, the state of the subject can be determined using the correlations between the measured moisture content and temperature.

Preferably, the moisture meter includes a main body, a measuring unit holder that is disposed at one end of the main body and is sandwiched in the armpit while holding the moisture measuring unit and the temperature measuring unit, and a display unit holder that is disposed at the other end of the main body so as to hold a display unit that displays the measured moisture content of the subject and the measured temperature of the subject.
According to this configuration, the main body has such a shape that the subject can easily hold or grip with the hand, the display unit holder can protrude to the front side from the armpit in a state where the measuring unit holder is sandwiched in the armpit, and the person who makes measurements can read the moisture content and temperature displayed on the display unit with the naked eyes.

Preferably, a plurality of the moisture measuring units and a plurality of the temperature measuring units are held on the measuring unit holder.
According to this configuration, since it is possible to obtain the average of the measured moisture content values using a plurality of moisture measuring units and to obtain the average of the measured temperature values using a plurality of temperature measuring units, it is possible to obtain more accurate moisture content and temperature.

Preferably, the moisture measuring unit emits light from a light emitting unit onto the moisture on a skin of the armpit to receive the light reflected from the moisture using a light receiving unit and measures the moisture content based on a change in an amount of the received light.
According to this configuration, it is possible to measure the moisture content in the armpit of the subject in an optical manner.

### Advantageous Effects of Invention

The present invention can provide a moisture meter that can easily measure a moisture content of a subject and can be effectively used as means for assisting the subject in appropriately regulating moisture content.

### Brief Description of Drawings

FIG. 1 is a diagram showing a state where a subject uses an embodiment of a moisture meter according to the present invention.
FIG. 2 is a diagram showing an appearance of the moisture meter shown in FIG. 1 from various directions.
FIG. 3 is a block diagram showing a functional configuration of the moisture meter shown in FIG. 2.
FIG. 4 is a diagram showing an example of the structure of an optical moisture measuring unit.
FIG. 5 is a diagram showing examples of patient symptoms based on correlations between a moisture content in the living body of a subject M and the temperature of the living body of the subject M.
FIG. 6 is a flowchart showing an example of a moisture content detecting operation of the moisture meter according to the present invention.
FIG. 7 is a diagram showing an appearance of another embodiment of the present invention from various directions.

### Description of Embodiments

Hereinafter, preferred embodiments of the present invention will be described with reference to the drawings.
The embodiments described below are specific preferred examples of the present invention, and various limitations that are technically preferable are added. However, the scope of the present invention is not limited to these embodiments unless there is a particular statement that restricts the present invention.

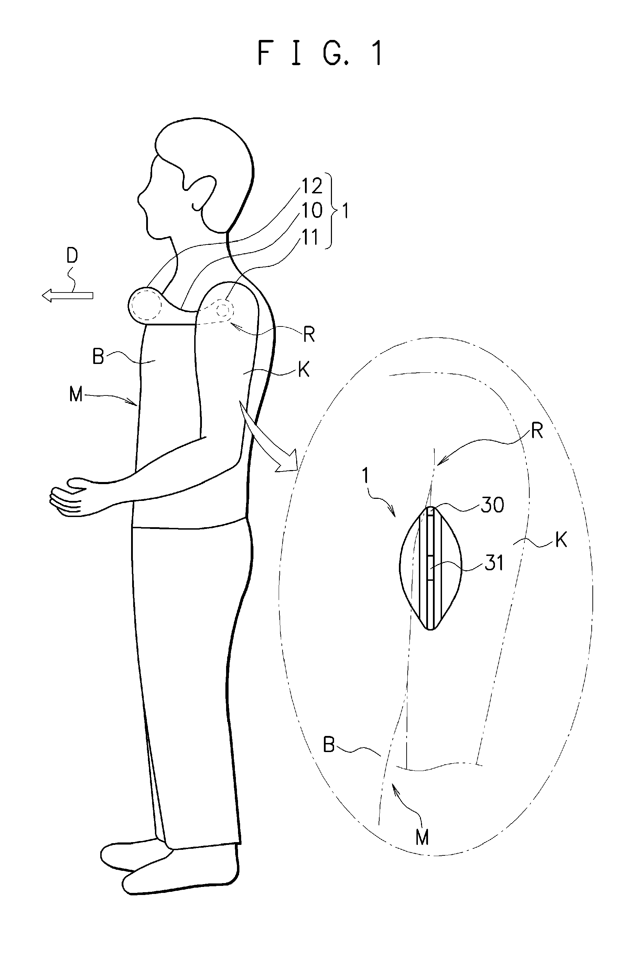

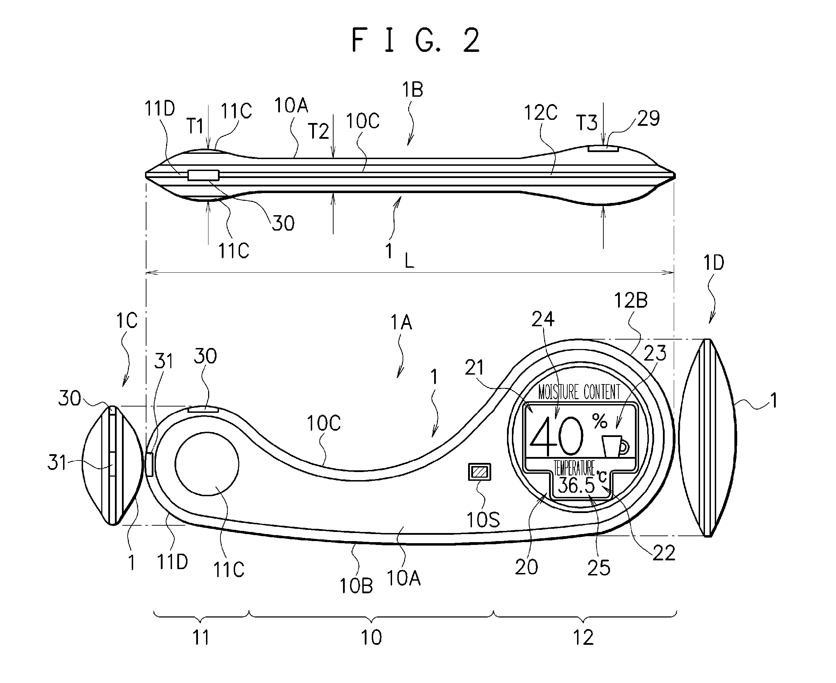

FIG. 1 is a diagram showing a state where a subject uses a preferred embodiment of a moisture meter according to the present invention. FIG. 2 is a diagram showing an example of an external structure of the moisture meter shown in FIG. 1.
A part 1A of a moisture meter 1 shown in FIG. 2 shows a front part of the moisture meter 1, a part 1B of the moisture meter 1 shows an upper part of the moisture meter 1, a part 1C of the moisture meter 1 shows a side part of the moisture meter 1 shown in the part 1A as seen from the left side of the figure, and a part 1D of the moisture meter 1 is a side part of the moisture meter 1 shown in the part 1A as seen from the right side of the figure.

The moisture meter 1 shown in FIGS. 1 and 2 may be an electronic moisture meter or an armpit-type electronic moisture meter, and the moisture meter 1 is a compact and portable moisture meter. As shown in FIG. 2, the moisture meter 1 roughly includes a main body 10, a measuring unit holder 11, and a display unit holder 12. A total weight of the moisture meter 1 is as light as approximately 20 grams, for example.
The main body 10, the measuring unit holder 11, and the display unit holder 12 are made from plastics, for example, and one end of the main body 10 is formed to be continuous with the measuring unit holder 11, and the other end of the main body 10 is formed to be continuous with the display unit holder 12.
The main body 10 is formed in such a shape that a subject M or the person who makes measurements can easily hold or grip. For example, the main body 10 includes a first curved portion 10B that is smoothly curved outward and a second curved portion 10C that is greatly curved inward, and the second curved portion 10C is more curved than the first curved portion 10B.

The reason why the main body 10 is formed in such a characteristic shape is to allow the subject M or the person who makes measurements to hold or grip the main body 10 with the hand to insert the measuring unit holder 11 of the moisture meter 1 in an armpit R so that the measuring unit holder 11 can be reliably held. The reason for selecting the armpit R as the location of the living body where the moisture content of the subject M can be appropriately measured using the moisture meter 1 and measuring the moisture content in the living body of the subject M is as follows. That is, the reason why the moisture content in the armpit R is measured is because the moisture content reflects a moisture state of the entire living body of the subject M. For example, even if the subject is old and thin, the measuring unit holder 11 of the moisture meter 1 can be reliably inserted and held in the armpit R between the body and the upper arm. Further, even if the subject is an infant, the measuring unit holder 11 can be reliably inserted and held in the armpit R.

The moisture meter 1 shown in FIG. 2 may have the following dimensions, for example. The main body 10 has a total length L of about 110 mm for large size (for adults), approximately 110 mm for medium size, and approximately 90 mm for small and large size (for infants). The moisture meter 1 generally has a flat shape except for part of the measuring unit holder 11 and the display unit holder 12.
The thickness T2 of a central portion 10A of the main body 10 is approximately 7 mm, the largest thickness T1 of the measuring unit holder 11 is approximately 9 mm, and the largest thickness T3 near the display unit holder 12 is approximately 14 mm.
However, these dimensions of the moisture meter 1 are not limited to the above examples but can be selected optionally. [0020] As shown in FIG. 2, the measuring unit holder 11 of the moisture meter 1 includes a circular peripheral portion 11D, and two convex portions 11C. When the measuring unit holder 11 is inserted in the armpit R of the subject M shown in FIG. 1 using the two convex portions 11C and held by being pressed by an upper arm K, the moisture content in the living body of the subject M and the temperature thereof can be measured stably.
In this manner, in a state where the measuring unit holder 11 of the moisture meter 1 is held in the armpit R, by bringing the main body 10 into close contact with a side portion of an upper body B of the subject, the moisture meter 1 can be more reliably held closer to the upper body B.
For example, as shown in FIG. 1, when the moisture meter 1 is used, the display unit holder 12 can be held approximately horizontally so as to face the front D of the subject M. The distance between the measuring unit holder 11 and the display unit holder 12, that is the length of the main body 10 is set such that, when the subject M inserts the measuring unit holder 11 in the armpit R, a display unit 20 in the display unit holder 12 is positioned at a position outside the armpit R (the position where the display unit 20 is not pinched between the body portion of the subject M and the upper arm K).

The display unit holder 12 shown in FIG. 2 includes a circular peripheral portion 12B, and the display unit 20 having a circular shape, for example, is held on the front side of the display unit holder 12. A liquid crystal display device, an organic EL device, and the like can be used as the display unit 20, for example. A speaker 29 as a sound generator is disposed on the back side of the display unit holder 12. In this manner, since the display unit 20 is disposed on the front side of the display unit holder 12, and the speaker 29 is disposed on the back side, the display unit 20 and the speaker 29 are not positioned in the armpit R. Thus, the subject M can easily check the moisture content and the temperature displayed on the display unit 20 and listen to sound guidance or the like generated from the speaker 29.

As shown in FIG. 2, the display unit 20 includes a screen (hereinafter referred to as a moisture content display screen) 21 for displaying the moisture content (%) in the living body of the subject and a screen (hereinafter referred to as a temperature display screen) 22 for displaying the temperature (°C). The moisture content display screen 21 includes a moisture content suggestive mark 23 and can display the moisture content using a relatively large digital indication 24 for example as 40%. In the example of FIG. 2, the temperature display screen 22 can display the temperature of the subject using a temperature digital indication 25 for temperature in a smaller size than the moisture content digital indication 24. However, the configuration of the display unit 20 is not limited to the example shown in FIG. 2, and the moisture content digital indication 24 and the temperature digital indication 25 may have the same size.

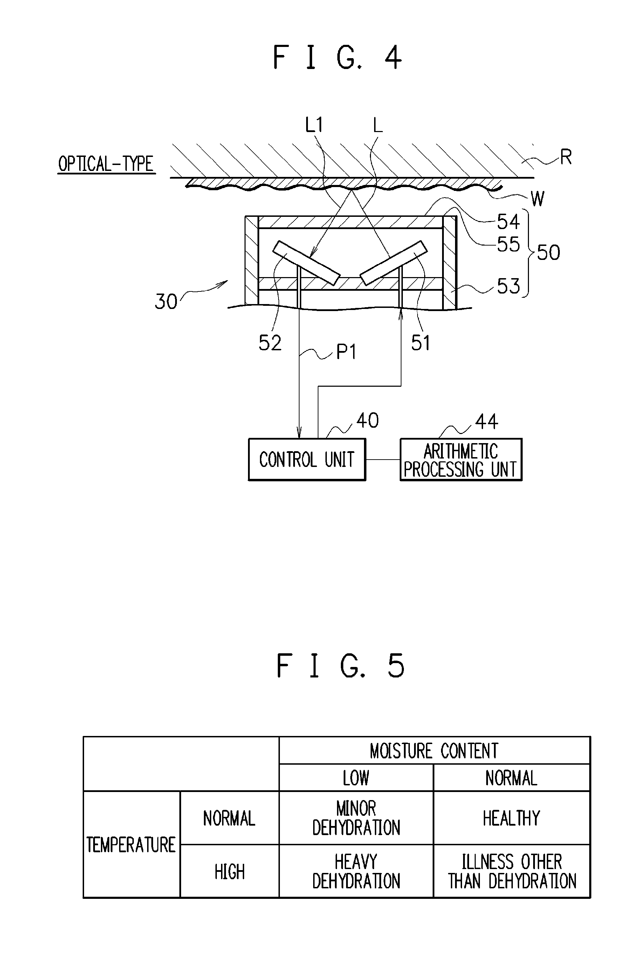

As shown in FIG. 2, the measuring unit holder 11 of the moisture meter 1 holds a probe-type moisture measuring unit 30 and a temperature measuring unit 31. Preferably, anti-slip means is arranged on the surface of the measuring unit holder 11 by forming an uneven surface according to dimple processing or the like, for example. According to this configuration, when the subject M inserts the measuring unit holder 11 in the armpit R, it is possible to provide such a shape that the measuring unit holder 11 of the moisture meter 1 is reliably and stably sandwiched and to decrease thermal capacity to attain a thermal equilibrium state early.
The probe-type moisture measuring unit 30 shown in FIG. 2 is a portion that measures, in the armpit R of the subject shown in FIG. 1, the moisture content in the living body of the subject M, and preferably, is disposed along the peripheral portion 11D of the measuring unit holder 11 so as to be exposed. Due to this, the moisture measuring unit 30 can be reliably in direct contact with the skin surface of the armpit R. Although the probe-type moisture measuring unit 30 can use various types such as optical-type, electric resistance-type, electrostatic capacitance-type, ultrasound-type, or absolute dry-type, the probe-type moisture measuring unit 30 may employ an optical measuring unit to measure the moisture content on the surface of the armpit R in an optical manner. An example of the structure of the moisture measuring unit 30 will be described later with reference to FIG. 4.
The temperature measuring unit 31 of FIG. 2 is a portion that measures the temperature of the living body of the subject M in the armpit R of the subject shown in FIG. 1, and preferably, is disposed along the peripheral portion 11D of the measuring unit holder 11 so as to be exposed.

Returning to FIG. 2, the temperature measuring unit 31 is a portion that measures the temperature of the living body in the armpit R of the subject, and preferably, is disposed along the peripheral portion 11D of the measuring unit holder 11 so as to be exposed. In this manner, the temperature measuring unit 31 can be reliably in direct contact with the skin surface of the armpit R.
The temperature measuring unit 31 is configured to detect the temperature by making contact with the armpit R of the subject M shown in FIG. 1, and for example, a temperature measuring unit having a thermistor or a thermocouple may be used as the temperature measuring unit 31. For example, a temperature signal detected by the thermistor is converted into a digital signal and is output. The thermistor is liquid-tightly protected by a metal cap made from stainless steel, for example.

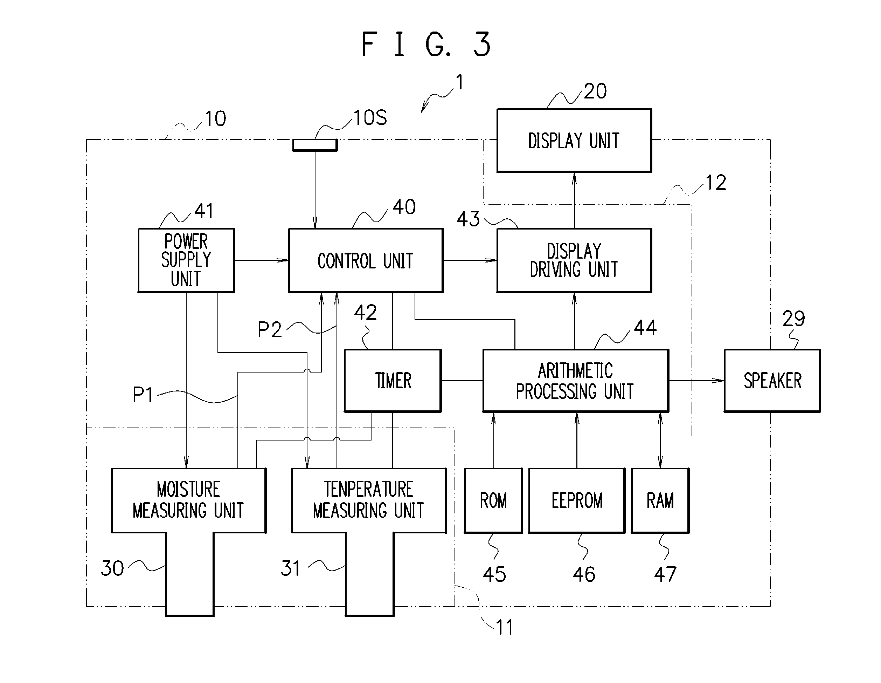

FIG. 3 is a block diagram showing a functional configuration of the moisture meter 1 shown in FIG. 2.
In the block diagram of the moisture meter 1 shown in FIG. 3, the main body 10 includes a control unit 40, a power supply unit 41, a timer 42, a display driving unit 43, an arithmetic processing unit 44, a read only memory (ROM) 45, an electrically erasable PROM (EEPROM) 46, and a random access memory (RAM) 47. The moisture measuring unit 30 and the temperature measuring unit 31 are disposed in the measuring unit holder 11, and the display unit 20 and the speaker 29 are disposed in the display unit holder 12.

The power supply unit 41 of FIG. 3 is a rechargeable secondary battery or a primary battery and supplies power to the control unit 40, the moisture measuring unit 30, and the temperature measuring unit 31. The control unit 40 is electrically connected to a power switch 10S, the moisture measuring unit 30, the temperature measuring unit 31, the timer 42, the display driving unit 43, and the arithmetic processing unit 44. The control unit 40 controls the entire operation of the moisture meter 1.
The display unit 20 of FIG. 3 is electrically connected to the display driving unit 43, and the display driving unit 43 displays the moisture content suggestive mark 23 such as a cup, the moisture content digital indication 24, and the temperature digital indication 25 as shown in FIG. 2 on the display unit 20 according to a command from the control unit 40.

The arithmetic processing unit 44 of FIG. 3 is electrically connected to the speaker 29, the ROM 45, the EEPROM 46, and the RAM 47. The ROM 45 stores a program for estimating and calculating the moisture content and temperature of the subject based on a change over time in moisture content data and temperature data, calculated from the moisture content data obtained from an impedance value measured by the moisture measuring unit 30 and the temperature data measured by the temperature measuring unit 31 based on the time measured by the timer 42. The EEPROM 46 stores predetermined audio data. The RAM 47 can store the calculated moisture content data and temperature data in association with time.
The arithmetic processing unit 44 estimates and calculates the moisture content and temperature of the subject according to the program stored in the ROM 45 and outputs audio data to the speaker 29.

Next, an example of the structure of the moisture measuring unit 30 will be described with reference to FIG. 4.
FIG. 4 shows an example of the structure of the optical moisture measuring unit 30.
The optical moisture measuring unit 30 shown in FIG. 4(A) includes a container portion 50, a light emitting unit 51, and a light receiving unit 52, and the container portion 50 stores the light emitting unit 51 and the light receiving unit 52. The container portion 50 has a non-transparent circumferential portion 53 made from a resin and a lid portion 54 made from an optically transparent resin, disposed in an opening 55 of the circumferential portion 53. Due to this, since the light emitting unit 51 and the light receiving unit 52 are liquid-tightly sealed by the container portion 50, when measuring the moisture content of the skin of the armpit R by bringing the lid portion 54 of the container portion 50 in close contact with the skin of the armpit, the moisture W on the skin of the armpit R is prevented from adhering to the light emitting unit 51 and the light receiving unit 52. Thus, it is possible to prevent a problem that the moisture W on the skin of the armpit R adheres to the light emitting unit 51 and the light receiving unit 52 when measuring the moisture content.

The light emitting unit 51 of FIG. 4(A) is configured to emit light L in the infrared region, for example, onto the skin of the armpit R through the lid portion 54 and receive reflected light L1 through the lid portion 54 using the light receiving unit 52. The optical moisture measuring unit 30 uses the fact that the larger the amount of the moisture W on the skin of the armpit R, the larger the amount of the light L absorbed in the moisture W and the smaller becomes the amount of the received light. The light receiving unit 52 detects the amount of the moisture W on the skin of the armpit R, the moisture content data signal P1 from the light receiving unit 52 is delivered to the control unit 40, and the arithmetic processing unit 44 calculates the moisture content based on the moisture content data signal P1.
In this manner, the optical moisture measuring unit 30 can emit the light L from the light emitting unit 51 onto the moisture on the skin of the armpit R to receive the reflected light L1 using the light receiving unit 31 to measure the moisture content based on a change in the amount of received light. Thus, it is possible to measure the moisture content in the armpit R of the subject in an optical manner.
In this manner, the arithmetic processing unit 44 estimates and calculates the moisture content and temperature of the subject based on a change over time of the moisture content data and temperature data of the subject, obtained from the moisture content data P1 measured by the moisture measuring unit 30 and the temperature data P2 measured by the temperature measuring unit 31.

The symptoms of a subject can be determined from the correlations between the moisture content in the living body of the subject M and the temperature of the living body of the subject M, for example, which will be described as specific examples of the symptoms of the subject with reference to FIG. 5.
The correlations between the moisture content in the living body of the subject M and the temperature of the living body of the subject M shown in FIG. 5 are stored in the ROM 45 of FIG. 3, for example.
In FIG. 5, it can be determined that when the moisture content is low and the temperature is normal, the subject has minor dehydration, whereas when the moisture content is normal and the temperature is normal, the subject is healthy. In contrast, it can be determined that when the moisture content is low and the temperature is high, the subject has severe dehydration, whereas when the moisture content is normal and the temperature is high, the subject has an illness other than dehydration such as a cold.

In this manner, since the health, minor or severe dehydration, cold-like symptoms of a subject can be determined from the moisture content and temperature of the living body of the subject, it is important for the moisture meter 1 according to the embodiment of the present invention to measure the moisture content and temperature in the armpit R. The determination results on the symptoms of the subject may be displayed on the display unit 20 shown in FIG. 2.

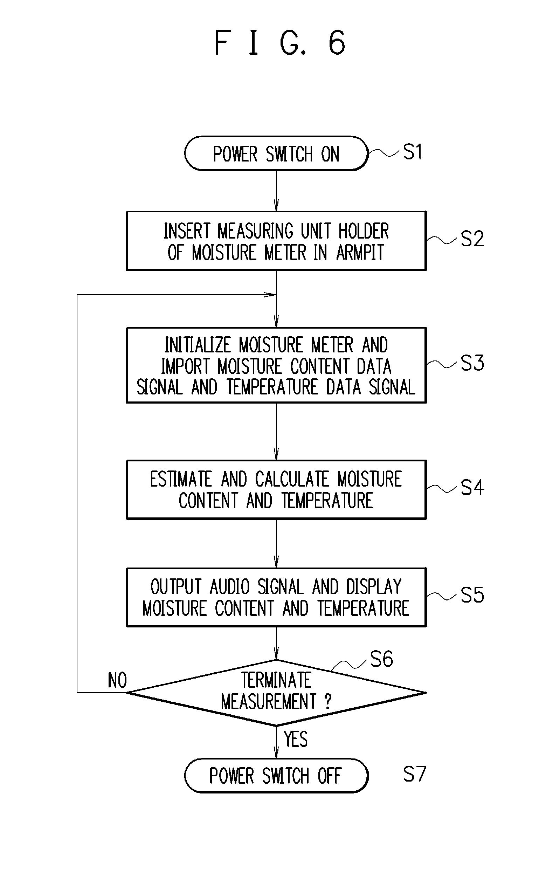

FIG. 6 is a flowchart showing an example of the operation of the moisture meter 1 detecting the moisture content and temperature of the subject M.
Next, an example of the operation of the moisture meter 1 shown in FIGS. 1 and 2, detecting the moisture content and temperature of the subject M will be described with reference to FIG. 6.
In step S1 of FIG. 6, the subject turns ON the power switch 10S shown in FIG. 3, and when the ON signal is delivered to the control unit 40, the moisture meter 1 enters a measurement ready state. In step S2, as shown in FIG. 1, the subject M inserts the measuring unit holder 11 of the moisture meter 1 in the armpit R using the two convex portions 11C shown in FIG. 2.

In a state where the measuring unit holder 11 of the moisture meter 1 is held in the armpit R, by bringing the main body 10 into closer contact with the side portion of the upper body B of the subject, the moisture meter 1 can be more reliably held on the upper body B of the subject. For example, the display unit holder 12 can be positioned approximately horizontally so as to face the front D of the subject M.
When the distance between the measuring unit holder 11 and the display unit holder 12 is set such that, when the subject M inserts the measuring unit holder 11 in the armpit R, the display unit 20 is positioned at a position outside the armpit R (the position where the display unit 20 is not pinched between the body portion and the upper arm). Thus, the subject M can easily read the moisture content digital indication 24 and the temperature digital indication 25 on the display unit 20 of the display unit holder 12. Further, the subject M can listen to the sound guidance generated from the speaker 29.

In step S3 of FIG. 6, when the measuring unit holder 11 of the moisture meter 1 is held in the armpit R, the arithmetic processing unit 44 initializes the moisture meter 1 and imports moisture content data signals P1 measured by the moisture measuring unit 30 and temperature data signals P2 measured by the temperature measuring unit 31 at predetermined sampling points in time based on a timing signal from the timer 42.
In step S4, the arithmetic processing unit 44 can estimate and calculate the moisture content and temperature of the subject based on a change over time of the moisture content data and the temperature data of the subject, obtained from the moisture content data P1 measured by the moisture measuring unit 30 and the temperature data P2 measured by the temperature measuring unit 31.

In step S5 of FIG. 6, the calculated values of the moisture content and temperature of the subject M can be output from the speaker 29 of FIG. 3 as audio guidance, and the relatively large digital indication 24 and the temperature digital indication 25 can be displayed on the moisture content display screen 21 and the temperature display screen 22 of the display unit 20 shown in FIGS. 3 and 2, respectively.
In step S6, when the subject M terminates the measurement using the moisture meter 1, the power switch 10S of FIG. 3 is turned off. However, when the subject M does not terminate the measurement, the flow returns to step S3, and the processes of steps S3 to S6 are repeated.

The moisture meter 1 according to the embodiment of the present invention has a structure that the moisture content of the subject M can be measured in the armpit R where the moisture content can be measured appropriately. As a result, the moisture meter 1 can be effectively used as means for assisting in regulating an appropriate moisture content of infants and older persons, who have difficulty in drinking water appropriately when feeling thirsty, or of normal persons, who are exercising vigorously, as well as assisting in regulating a moisture content that is extremely vital to maintaining health in daily life.
The reason for selecting the armpit R as the location of the living body where the moisture content of the subject M can be appropriately measured and measuring the moisture content in the armpit R is because the moisture content in the armpit R reflects the moisture state of the entire living body of the subject M. In general, the skins of older persons are easily to dry, and the degree thereof varies greatly from person to person. Among the skins, the armpit R is less influenced from the outside as compared to other locations and incurs a small variation in measurement and is thus suitable for measurement. Even if the subject is old and thin, the measuring unit holder 11 of the moisture meter 1 can be reliably inserted and held in the armpit R between the body and the upper arm. Further, even if the subject is an infant, the measuring unit holder 11 can be reliably inserted and held in the armpit R. Furthermore, since the moisture measuring unit 30 has such a structure that it secures the central portion of the armpit R and thus provides higher measurement accuracy.

The moisture meter 1 according to the embodiment of the present invention preferably has such a structure that it can also measure the temperature of the armpit R simultaneously with measuring appropriately the moisture content of the subject M in this manner. Due to this, as shown in FIG. 5, health workers or caregivers can measure the moisture content of the subject M more easily since they only need to hold the measuring unit holder 11 of the moisture meter 1 in the armpit R of the subject M than measuring the moisture content from the mouth or the like.
As shown in FIG. 2, from the correlations between the moisture content in the living body of the subject M and the temperature of the living body of the subject M displayed on the display unit 20, it can be determined that when the moisture content is low and the temperature is normal, the subject has minor dehydration, whereas when the moisture content is normal and the temperature is normal, the subject is healthy. In contrast, it can be roughly determined by the doctor that when the moisture content is low and the temperature is high, the subject has severe dehydration, whereas when the moisture content is normal and the temperature is high, the subject has an illness other than dehydration such as a cold.

The embodiment of the moisture meter according to the present invention is a moisture meter for measuring a moisture content of a subject, including: a probe-type moisture measuring unit that is held in an armpit of the subject so as to measure a moisture content in the armpit in order to measure the moisture content of the subject. Due to this, the moisture meter can easily measure the moisture content of the subject and can be used effectively as means for assisting the subject in regulating an appropriate moisture content. The reason for selecting the armpit as the location of the living body where the moisture content of the subject can be appropriately measured using the moisture meter and measuring the moisture content in the armpit R is because the moisture content in the armpit R reflects the moisture state of the entire living body of the subject M.

In the embodiment of the moisture meter according to the present invention, the moisture meter includes a temperature measuring unit that is held in the armpit of the subject so as to measure the temperature of the subject. According to this configuration, by measuring the temperature of the subject simultaneously with measuring the moisture content of the subject in the armpit of the subject, the state of the subject can be determined using the correlations between the measured moisture content and temperature.

In the embodiment of the moisture meter according to the present invention, the moisture meter includes a main body, a measuring unit holder that is disposed at one end of the main body and is sandwiched in the armpit while holding the moisture measuring unit and the temperature measuring unit, and a display unit holder that is disposed at the other end of the main body so as to hold a display unit that displays the measured moisture content of the subject and the measured temperature of the subject. According to this configuration, the main body has such a shape that the subject M can easily hold or grip with the hand, the display unit holder can protrude to the front side from the armpit in a state where the measuring unit holder is sandwiched in the armpit, and the person who makes measurements can read the moisture content and temperature displayed on the display unit with the naked eyes.

In the embodiment of the moisture meter according to the present invention, a plurality of the moisture measuring units and a plurality of the temperature measuring units are held on the measuring unit holder. According to this configuration, since it is possible to obtain the average of the measured moisture content values using a plurality of moisture measuring units and to obtain the average of the measured temperature values using a plurality of temperature measuring units, it is possible to obtain more accurate moisture content and temperature.

In the embodiment of the moisture meter according to the present invention, the moisture measuring unit emits light from a light emitting unit onto the moisture on the skin of the armpit to receive the light reflected from the moisture and measures the moisture content based on a change in an amount of the received light. According to this configuration, it is possible to measure the moisture content in the armpit of the subject in an optical manner.

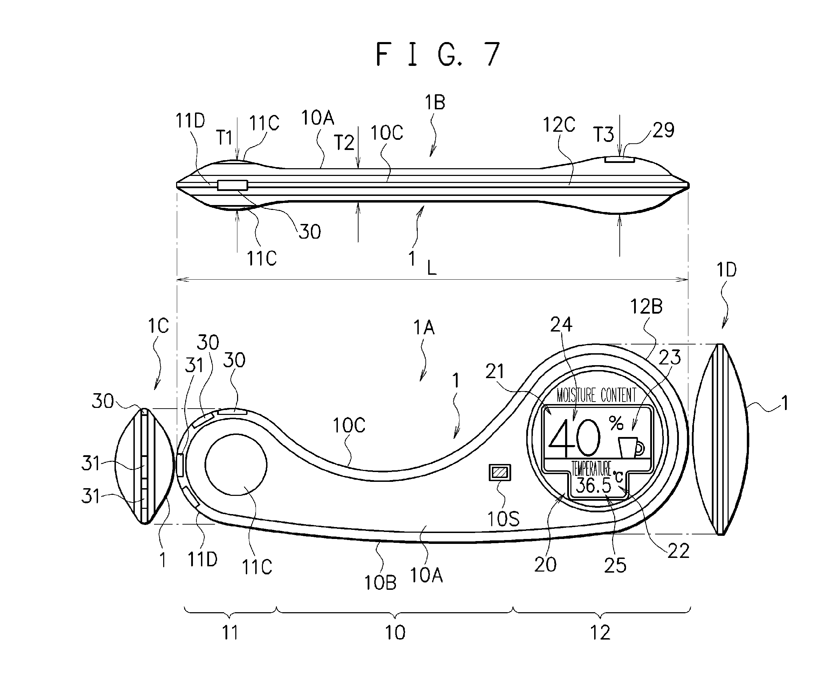

The present invention is not limited to the above embodiment. Various changes can be made to the present invention, and various modifications can be made within the scope described in the claims.
In the illustrated example, one probe-type moisture measuring unit 30 and one temperature measuring unit 31 are disposed in the measuring unit holder 11.
However, the present invention is not limited to this, and as shown in FIG. 7, a plurality of probe-type moisture measuring units 30 and a plurality of temperature measuring units 31 may be disposed in the measuring unit holder 11. According to this configuration, it is possible to further improve moisture content measurement accuracy by averaging the moisture content values obtained by the moisture measuring units 30 and to further improve temperature measurement accuracy by averaging the temperature values obtained by the temperature measuring units 31. Moreover, only one of the moisture measuring unit 30 and the temperature measuring unit 31 may be provided plurally in the measuring unit holder 11.

The probe-type moisture measuring unit may employ moisture content measurement that uses near-infrared spectroscopy. In this case, light in the near-infrared region is emitted onto the face or the position of the skin to be measured to measure reflected spectrum to measure a moisture content in the stratum corneum of the skin.
Specific wavelengths of light absorbed by moisture include 1.2 µm, 1.45 µm, 1.94 µm, and 2.95 µm, and any one of these wavelengths may be used. The use of the measurement light only may cause a variation in measurement results due to the influence of a measurement distance, the color of the object to be measured, the surface state, and the like. Thus, light that does not affect the moisture adjacent to the measurement light may be simultaneously measured as reference light. The measurement light and the reference light are influenced by approximately the same extend by a variation in measurement distance, the color, and the surface state. Thus, it is preferable to use both of them because the influence of disturbance can be eliminated by calculating the ratio between them.
The probe-type moisture measuring unit may employ moisture content measurement that uses spectrometric thermal analysis. In this case, dispersed light is emitted onto a measurement target object, a variation in the temperature resulting from optical absorption in the measurement target object is detected by a temperature sensor, and the moisture content in the measurement target object is calculated.
When electromagnetic waves are used, the apparatus may become too large.

### Reference Sighs List

1: MOISTURE METER
10: MAIN BODY
11: MEASURING UNIT HOLDER
12: DISPLAY UNIT
M: SUBJECT
R: ARMPIT
11: MEASURING UNIT HOLDER
12: DISPLAY UNIT HOLDER
12: DISPLAY UNIT HOLDER
20: DISPLAY UNIT
30: PROBE-TYPE MOISTURE MEASURING UNIT
31: TEMPERATURE MEASURING UNIT

## Claims

1. A moisture meter for measuring a moisture content of a subject, comprising:
a probe-type moisture measuring unit that is held in an armpit of the subject so as to measure a moisture content in the armpit in order to measure the moisture content of the subject.

2. The moisture meter according to claim 1, further comprising:
a temperature measuring unit that is held in the armpit of the subject so as to measure temperature of the subject.

3. The moisture meter according to claim 2, further comprising:
a main body;
a measuring unit holder that is disposed at one end of the main body and is sandwiched in the armpit while holding the moisture measuring unit and the temperature measuring unit; and
a display unit holder that is disposed at the other end of the main body so as to hold a display unit that displays the measured moisture content of the subject and the measured temperature of the subject.

4. The moisture meter according to claim 3, wherein
a plurality of the moisture measuring units and a plurality of the temperature measuring units are held on the measuring unit holder.

5. The moisture meter according to any one of claims 1 to 4, wherein
the moisture measuring unit emits light from a light emitting unit onto the moisture on a skin of the armpit to receive the light reflected from the moisture using a light receiving unit and measures the moisture content based on a change in an amount of the received light.
